(19) Europäisches Patentamt
European Patent Office
Office européen des brevets

(11) EP 0 636 116 B1

(12) **EUROPÄISCHE PATENTSCHRIFT**

(45) Veröffentlichungstag und Bekanntmachung des Hinweises auf die Patenterteilung:
**13.08.1997 Patentblatt 1997/33**

(21) Anmeldenummer: **93909367.0**

(22) Anmeldetag: **13.04.1993**

(51) Int Cl.[6]: **C07C 49/21**, C07C 33/05, C07C 47/225, A61K 7/46

(86) Internationale Anmeldenummer:
**PCT/EP93/00883**

(87) Internationale Veröffentlichungsnummer:
**WO 93/21142 (28.10.1993 Gazette 1993/26)**

# (54) PENTEN-DERIVATE, DEREN HERSTELLUNG UND VERWENDUNG

PENTENE DERIVATIVES, THEIR PREPARATION AND THEIR USE

DERIVES DE PENTENE, LEUR FABRICATION ET UTILISATION

(84) Benannte Vertragsstaaten:
**AT BE CH DE ES FR GB GR IT LI NL**

(30) Priorität: **18.04.1992 DE 4212941**

(43) Veröffentlichungstag der Anmeldung:
**01.02.1995 Patentblatt 1995/05**

(73) Patentinhaber: **Henkel Kommanditgesellschaft auf Aktien**
**40191 Düsseldorf (DE)**

(72) Erfinder:
- **MARKERT, Thomas**
  **D-4000 Düsseldorf (DE)**
- **PORRMANN, Volker**
  **D-4010 Hilden (DE)**
- **BRUNS, Klaus**
  **D-4150 Krefeld-Traar (DE)**

(56) Entgegenhaltungen:
**EP-A- 0 155 591**
**EP-A- 0 466 019**
**DD-A- 266 347**
**DE-B- 2 513 996**
**US-A- 3 903 174**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 636 116 B1

## Beschreibung

Die Erfindung betrifft neue Penten-Derivate, ein Verfahren zu deren Herstellung sowie deren Verwendung als Riechstoffe.

## Stand der Technik

Viele natürliche Riechstoffe stehen, gemessen am Bedarf, in völlig unzureichender Menge zur Verfügung. Aus parfümistischer Sicht besonders geschätzt und wertvoll ist das Sandelholzöl. Es wird durch Wasserdampfdestillation aus dem Kernholz des Sandelbaumes gewonnen, eines tropischen Halbparasiten, der in Indien und Malaysia vorkommt. Kernholz erscheint nach etwa zehn Jahren und beginnt erst bei zwanzigjährigen Bäumen, sich rascher auszubilden. Voll ausgewachsene Bäume werden im Alter von 30 bis 60 Jahren ausgerodet, da die Wurzeln besonders reich an wohlriechendem Kernholz sind [vergl. E.T.Morris, Dragoco Report 1983 (30), 40]. Es ist daher verständlich, daß die Riechstoff-Forschung ständig bemüht ist, geeignete Substitute für natürliches Sandelholzöl zu entwickeln.

Die Schwerpunkte bei der Entwicklung geeigneter Substitute für natürliches Sandelholzöl hat R.E.Naipawer in einem Review skizziert [in: B.M.Lawrence, B.D.Mookherjee, B.J.Willis (Hrsg.): "Flavors and Fragrances: A World Perspective"; Elsevier Publishers, Amsterdam 1988]. Aus der europäischen Patentschrift EP 155 591 B1 sind Alkohole mit einer 2,2,3-Trimethyl-3-cyclopentenylgruppe bekannt, dabei unter anderem 2-Methyl-4-(2,2,3-trimethyl-3-cyclopenten-1-yl)-4-penten-1-ol.

Aus dem geschilderten Kontext heraus ist klar, daß die Riechstoffindustrie einen ständigen Bedarf an neuen Riechstoffen mit interessanten Duftnoten hat, um die Palette der natürlich verfügbaren Riechstoffe zu ergänzen und die notwendigen Anpassungen an wechselnde modische Geschmacksrichtungen vornehmen sowie den ständig steigenden Bedarf an Geruchsverbesserern für Produkte des täglichen Bedarfs wie Kosmetika und Reinigungsmittel decken zu können.

Darüber hinaus besteht generell ein ständiger Bedarf an synthetischen Riechstoffen, die sich günstig und mit gleichbleibender Qualität herstellen lassen und erwünschte olfaktorische Eigenschaften haben, d.h. angenehme, möglichst naturnahe und qualitativ neuartige Geruchsprofile von ausreichender Intensität besitzen und in der Lage sind, den Duft von kosmetischen und Verbrauchsgütern vorteilhaft zu beeinflussen. Es wurde daher nach Verbindungen gesucht, die charakteristische neue Geruchsprofile bei gleichzeitig hoher Haftfestigkeit, Geruchsintensität und Strahlkraft aufweisen sollten.

## Beschreibung der Erfindung

Es wurde nun gefunden, daß die Verbindungen der allgemeinen Formel (I) die oben genannten Forderungen in jeder Hinsicht ausgezeichnet erfüllen und in vorteilhafter Weise als Riechstoffe mit unterschiedlich nuancierten Geruchsnoten mit guter Haftfestigkeit eingesetzt werden können. Insbesondere wurde gefunden, daß die Verbindungen der Formel (I) gegenüber Verbindungen des Standes der Technik, die ihnen strukturell nahestehen, über eine verbesserte Geruchsintensität aufweisen, d.h. ihre Wirkung bereits bei niedrigeren Konzentrationen entfalten.

Gegenstand der vorliegenden Erfindung sind daher Penten-Derivate der allgemeinen Formel (I)

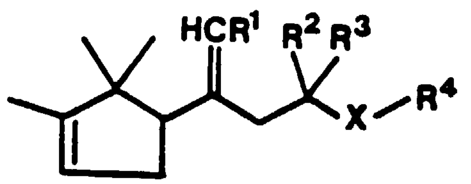

(I)

worin unabhängig voneinander der Rest $R^1$ Wasserstoff oder eine Methylgruppe, die Reste $R^2$ und $R^3$ eine Alkylgruppe mit 1 bis 5 C-Atomen, der Rest $R^4$ Wasserstoff oder eine Gruppe $CHR^5R^6$, die Reste $R^5$ und $R^6$ Wasserstoff oder eine Alkylgruppe mit 1 bis 6 C-Atomen und X eine Gruppe CO oder eine Gruppe CHOH bedeuten.

Die erfindungsgemäßen Verbindungen zeichnen sich durch eine verbesserte Geruchs-Intensität gegenüber den entsprechenden Derivaten aus, bei denen die Reste $R^2$ und $R^3$ Wasserstoff bedeuten. Insbesondere sind diejenigen Verbindungen (I) bevorzugt, bei denen die Reste $R^2$ und $R^3$ Methyl oder Ethyl bedeuten. Von diesen Verbindungen sind besonders bevorzugt:

(a) 3,3-Dimethyl-5-(2,2,3-trimethyl-3-cyclopenten-1-yl)-5-hexen-2-on,
(b) 3,3-Dimethyl-5-(2,2,3-trimethyl-3-cyclopenten-1-yl)-5-hexen-2-ol,

(c) 2,2-Dimethyl-4-(2,2,3-trimethyl-3-cyclopenten-1-yl)-4-penten-1-al,
(d) 2,2-Dimethyl-4-(2,2,3-trimethyl-3-cyclopenten-1-yl)-4-penten-1-ol.

Ganz besonders bevorzugt im Sinne der vorliegenden Erfindung sind diejenigen Penten-Derivate (I), bei denen die Reste $R^2$ und $R^3$ - unabhängig voneinander - Methyl- oder Ethylreste bedeuten.

In einer besonderen Ausführungsform der Erfindung hat $R^4$ die Bedeutung Wasserstoff, in einer anderen die Bedeutung Methyl.

Die Herstellung der neuen Verbindungen (I) erfolgt nach an sich bekannten Syntheseverfahren der organischen Chemie. Exemplarisch seien zwei besonders attraktive Zugangsmöglichkeiten dargestellt:

In einer ersten Variante wird der Allylalkohol 2-(2,2,3-Trimethyl-3-cyclopentenyl)-2-propen-1-ol durch Carroll-Reaktion zu Ketonen der allgemeinen Formel (I) umgesetzt, worin X eine Carbonylgruppe bedeutet. Unter Carroll-Reaktion wird dabei die Umlagerung von Allylalkoholen zu gamma-delta-ungesättigten Ketonen verstanden. Der Allylalkohol wird z.B. durch Umsetzung mit Acetessigester in den Acetessigsäureallylester überführt, der durch [3,3]-sigmatrope Umlagerung (Claisen-Umlagerung) zur alpha-Allylacetessigsäure und deren nachfolgende thermische Decarboxylierung in das gewünschte gamma-delta-ungesättigte Keton überführt wird. Der Acetessigsäureallylester kann dabei in Substanz eingesetzt oder in situ gebildet werden. Alternativ kann der Allyl alkohol mit Methyl-isoproypenylether - gewünschtenfalls in situ - in den entsprechenden Allylvinylether überführt werden, der der weiteren Umlagerung zu den Ketonen (I) ebenso wie der Acetessigsäureallylester zugänglich ist.

Die so erhaltenen Ketone (I) können anschließend in üblicher Weise an den der Ketogruppe benachbarten C-Atomen alkyliert werden, beispielsweise mit Methyljodid. Die Menge des eingesetzten Alkylierungsmittels richtet sich danach, welchen Grad der Alkylierung man einstellen will. Üblicherweise wird man pro Mol Carbonylverbindung 1 bis 5 mol, vorzugsweise 1 bis 3 mol Alkylierungsmittel und katalytische Mengen eines Phasentransferkatalysators einsetzen. In der Regel wird dabei ein Gemisch von im wesentlichen Mono-, Di- und Trialkylierungsprodukten als Rohprodukt erhalten, aus denen die einzelnen Individuen (I) nach üblichen Methoden, zum Beispiel durch Destillation, isoliert werden können.

Die Carbonylfunktion der alkylierten Carbonylverbindungen läßt sich in einer weiteren Reaktion zur OH-Gruppe reduzieren, zum Beispiel mittels komplexer Hydride wie Lithiumaluminiumhydrid oder Lithium- bzw. Natriumborhydrid. Auf diese Weise werden weitere Penten-Derivate der allgemeinen Formel (I) erhalten.

In einer zweiten Variante werden zur Herstellung derjenigen Verbindungen (I), bei denen X eine Carbonylgruppe und der Rest $R^4$ Wasserstoff bedeutet Allylalkohole der allgemeinen Formel (II)

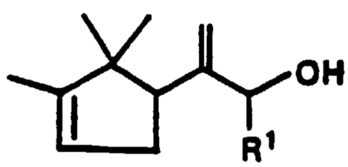

(II)

worin der Rest $R^1$ Wasserstoff oder eine Methylgruppe bedeutet, mit Aldehyden der allgemeinen Formel (III)

$$CHR^2R^3\text{-}CHO \qquad \text{(III)}$$

worin unabhängig voneinander die Reste $R^2$ und $R^3$ Wasserstoff oder eine Alkylgruppe mit 1 bis 5 C-Atomen bedeuten, mit der Maßgabe, daß mindestens einer der Reste $R^2$ oder $R^3$ eine Alkylgruppe ist, umgesetzt. Bei dieser Reaktion entsteht aus den Verbindungen (II) und (III) zunächst intermediär ein Acetal, das nach Abspaltung eines Moleküls Alkohol - ebenfalls intermediär - in einen Vinylallylether übergeht, der in einer [3,3]-sigmatropen Umlagerung nach Claisen zu der entsprechenden erfindungsgemäßen Verbindung (I) führt. K.C. Brannock hat diesen Reaktionstyp ausgehend von speziellen Allylalkoholen und Isobutyraldehyd in J.Am.Chem.Soc. 1959, 81, 3379 beschrieben. Die Umsetzung des Allylalkohols mit dem Aldehyd wird in der Regel in Gegenwart einer katalytisch wirksamen Menge einer Säure, z.B. einer Sulfonsäure, Carbonsäure oder Lewis-Säure, durchgeführt.

Die nach dieser zweiten Variante zugänglichen Aldehyde können selbstverständlich weiter variiert werden. So erhält man durch Reduktion ihrer Carbonylfunktion zur OH-Gruppe, zum Beispiel mittels komplexer Hydride wie Lithiumaluminiumhydrid oder Lithium- bzw. Natriumborhydrid, weitere Penten-Derivate der allgemeinen Formel (I). Schließlich können die Aldehyde durch Umsetzung mit den entsprechenden Grignard-Reagentien zu weiteren Penten-Derivaten (I) umgesetzt werden.

Ein weiterer Gegenstand der Erfindung ist daher ein Verfahren zur Herstellung von Penten-Derivaten der allgemeinen Formel (I),

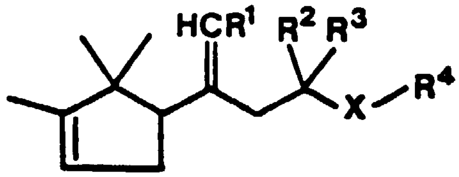

(I)

worin unabhängig voneinander der Rest $R^1$ Wasserstoff oder eine Methylgruppe, die Reste $R^2$ und $R^3$ eine Alkylgruppe mit 1 bis 5 C-Atomen, der Rest $R^4$ Wasserstoff oder eine Gruppe $CHR^5R^6$, die Reste $R^5$ und $R^6$ Wasserstoff oder eine Alkylgruppe mit 1 bis 6 C-Atomen und X eine Gruppe CO oder eine Gruppe CHOH bedeuten, durch

(a) Umsetzung von Allylalkoholen der allgemeinen Formel (II)

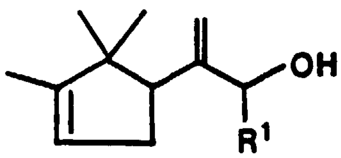

(II)

worin der Rest $R^1$ Wasserstoff oder eine Methylgruppe bedeutet, mit Aldehyden der allgemeinen Formel (III)

$$CHR^2R^3\text{-}CHO \qquad (III)$$

worin unabhängig voneinander die Reste $R^2$ und $R^3$ eine Alkylgruppe mit 1 bis 5 C-Atomen bedeuten,
oder
(b) Alkylierung von Verbindungen der allgemeinen Formel (IV)

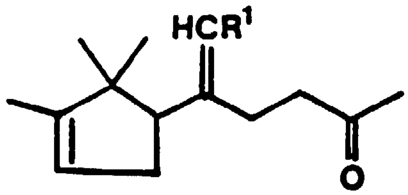

(IV)

worin der Rest $R^1$ Wasserstoff oder eine Methylgruppe bedeutet,
und jeweils gegebenenfalls anschließende Reduktion der Carbonylgruppe zur OH-Funktion, z.B. mittels komplexer Hydride.

Die erfindungsgemäßen Verbindungen (I) verfügen über bemerkenswerte Geruchseigenschaften. Ein weiterer Gegenstand der Erfindung ist daher die verwendung der Verbindungen der allgemeinen Formel (I) als Riechstoffe.

Dabei zeichnen sich die Verbindungen (I), in denen X eine Gruppe CO bedeutet, durch einen holzigen Geruch aus, der durch frische, fruchtige Aspekte nach Bergamotte und Rhabarber an Intensität gewinnt. Der gleiche Effekt tritt auch bei den aus diesen Ketonen durch Reduktion hergestellten Alkoholen auf, d.h. bei denjenigen Verbindungen (I), bei denen X eine Gruppe CHOH bedeutet, wobei hier die Geruchscharakteristik eine starke Sandelholznote mit fruchtigen Aspekten ist. Insbesondere zeichnen sich die Verbindungen, bei denen die Reste $R^2$ und $R^3$ Alkylreste sind, durch eine wesentlich höhere Geruchsintensität im Vergleich zu den entsprechenden Verbindungen, bei denen die Reste $R^2$ und/oder $R^3$ Wasserstoff bedeuten, aus, wobei die neuen Verbindungen ihre Sandelnote schon bei niedrigeren Dosierungen in der Kopfnote von Formulierungen entwickeln.

In Parfüm-Kompositionen verstärken die Verbindungen (I) die Harmonie und Ausstrahlung sowie auch die Haftung, wobei die Dosierung unter Berücksichtigung der übrigen Bestandteile der Komposition auf die jeweils angestrebte Duftnote abgestimmt wird.

Daß die Penten-Derivate (I) Sandel-Noten aufweisen, war nicht vorhersehbar und ist damit eine weitere Bestätigung für die allgemeine Erfahrung, daß die olfaktorischen Eigenschaften bekannter Riechstoffe keine zwingenden Rückschlüsse auf die Eigenschaften strukturverwandter Verbindungen zulassen, weil weder der Mechanismus der Duftwahrnehmung noch der Einfluß der chemischen Struktur auf die Duftwahrnehmung hinreichend erforscht sind, somit also normalerweise nicht vorhergesehen werden kann, ob ein geänderter Aufbau bekannter Riechstoffe überhaupt zur Änderung der olfaktorischen Eigenschaften führt und ob diese Änderungen positiv oder negativ beurteilt werden.

Die Verbindungen der Formel (I) eignen sich aufgrund ihres Geruchsprofils insbesondere auch zur Modifizierung und Verstärkung bekannter Kompositionen. Hervorgehoben werden soll insbesondere ihre außerordentliche Geruchsstärke, die ganz allgemein zur Veredelung der Komposition beiträgt.

Die Verbindungen der Formel (I) lassen sich mit zahlreichen bekannten Riechstoffingredientien, z.B. anderen Riechstoffen natürlichen, synthetischen oder partial-synthetischen Ursprungs, etherischen Ölen und Pflanzenextrakten kombinieren. Die Palette der natürlichen Riechstoffe kann dabei sowohl leicht- als auch mittel- und schwerflüchtige Komponenten und diejenige der synthetischen Riechstoffe Vertreter aus praktisch allen Stoffklassen umfassen. Beispiele sind:

(a) <u>Naturprodukte</u> wie Baummoos-Absolue, Basilikumöl, Agrumenöle wie Bergamotteöl, Mandarinenöl, usw., Mastix-Absolue, Myrtenöl, Palmarosaöl, Patchouliöl, Petitgrainöl, Wermutöl,Myrrheöl, Olibanumöl

(b) <u>Alkohole</u> wie Farnesol, Geraniol, Linalool, Nerol, Phenylethylalkohol, Rhodinol, Zimtalkohol, Sandalore [3-Methyl-5-(2.2.3-trimethylcyclopent-3-en-1-yl)pentan-2-ol], Sandela [3-Isocamphyl-(5)-cyclohexanol],

(c) <u>Aldehyde</u> wie Citral, Helional$^R$, $\alpha$-Hexylzimtaldehyd, Hydroxycitronellal, Lilial$^R$ [p-tert.-Butyl-$\alpha$-methyldihydrozimtaldehyd], Methylnonylacetaldehyd,

(d) <u>Ketone</u> wie Allylionon, $\alpha$-Ionon, $\beta$-Ionon, Isoraldein, Methylionon,

(e) <u>Ester</u> wie Allylphenoxyacetat, Benzylsalicylat, Cinnamylpropionat, Citronellylacetat, Citronellylethoxylat, Decylacetat, Dimethylbenzylcarbinylacetat, Ethylacetoacetat, Hexenylisobutyrat, Linalylacetat, Methyldihydrojasmonat, Vetiverylacetat, Cyclohexylsalicylat

(f) <u>Lactone</u> wie gamma-Undecalacton, 1-Oxaspiro[4.4]nonan-2-on, sowie verschiedene weitere in der Parfümerie oft benutzte Komponenten wie Ketonmoschus, Indol, p-Menthan-8-thiol-3-on, Methyleugenol, Ambroxan.

Bemerkenswert ist ferner die Art und Weise, wie die Verbindungen der Struktur (I) die Geruchsnoten einer breiten Palette bekannter Kompositionen abrunden und harmonisieren, ohne aber in unangenehmer Weise zu dominieren. Diejenigen Penten-Derivate (I), bei denen beide Reste $R^2$ und $R^3$ Alkylgruppen bedeuten, sind in dieser Hinsicht ganz besonders hervorzuheben.

Die erfindungsgemäßen Verbindungen enthalten Chiralitätszentren, so daß diese Verbindungen in verschiedenen Raumformen existieren können. Im Rahmen üblicher Synthesen fallen die erfindungsgemäßen Verbindungen als Gemische der entsprechenden Isomeren an und werden als solche als Riechstoffe verwendet.

Die einsetzbaren Anteile der erfindungsgemäßen Verbindungen oder deren Gemische in Riechstoffkompositionen bewegen sich von 1 bis 70 Gewichtsprozent, bezogen auf die gesamte Mischung. Gemische der erfindungsgemäßen Verbindungen (I) sowie Kompositionen dieser Art können sowohl zur Parfümierung kosmetischer Präparate wie Lotionen, Cremes, Shampoos, Seifen, Salben, Puder, Aerosole, Zahnpasten, Mundwässer, Desodorantien als auch in der alkoholischen Parfümerie (z.B. Eaux de Cologne, Eaux de Toilette, Extraits) verwendet werden. Ebenso besteht eine Einsatzmöglichkeit zur Parfümierung technischer Produkte wie Wasch- und Reinigungsmittel, Weichspüler und Textilbehandlungsmittel oder Tabak. Zur Parfümierung dieser verschiedenen Produkte werden diesen die Kompositionen in einer olfaktorisch wirksamen Menge, insbesondere in einer Konzentration von 0,05 bis 2 Gewichtsprozent, bezogen auf das gesamte Produkt, zugesetzt. Diese Werte sollen jedoch keine Grenzwerte darstellen, da der erfahrene Parfümeur auch mit noch geringeren Konzentrationen Effekte erzielen oder aber mit noch höheren Dosierungen neuartige Komplexe aufbauen kann.

Die folgenden Beispiele sollen den Gegenstand der Erfindung erläutern und sind nicht einschränkend aufzufassen.

Beispiele

1. Herstellung der Vorstufen für die erfindungsgemäßen Verbindungen

Beispiel 1: 5-(2,2,3-Trimethyl-3-cyclopenten-1-yl)-5-hexen-2-on

Ausführung: In einem 500 ml Dreihalskolben wurden 110 g (0,66 mol) 2-(2,2,3-Trimethyl-3-cyclopentenyl)-2-propenol (hergestellt aus α-Campholenaldehyd durch Kondensation mit Formaldehyd und Reduktion mit Lithiumaluminiumhydrid) mit 172,3 g Acetessigsäureethylester (1,33 mol) gemischt und 1,5 g N,N-Dimethylaminopyridin zugesetzt. Die Mischung wurde unter Rühren zwei Stunden lang auf Rückflußtemperatur erhitzt und das bei der Umesterung freiwerdende Ethanol wurde abdestilliert. Anschließend wurde unter Entfernung des freiwerdenden Ethanols die Temperatur auf 200 °C erhöht und sechs Stunden erhitzt. Zur Vervollständigung der Reaktion wurden weitere 100 g Acetessigester zugefügt und noch vierzehn Stunden auf 200 °C erhitzt.

Aufarbeitung: Das Reaktionsprodukt wurde durch Destillation an einer Vigreux-Kolonne und an einer Drehband-Kolonne gereinigt. Die Hauptmenge des Destillats wurde bei Kopftemperaturen von 127-135 °/15 mbar mit einer GC-Reinheit von 98 % erhalten. Die Ausbeute betrug 14 % der Theorie.

Charakterisierung: Das IR-Spektrum des Produkts (Film auf NaCl) zeigte Absorptionsbanden bei 2955, 1718 (C=O), 1639 (C=C), 1358 und 1160 $cm^{-1}$. Geruch: Linalyacetat-, Bergamotte-, Basilikum-Note, blumig, holzig.

2. Herstellung der erfindungsgemäßen Verbindungen

Beispiel 2: 3,3-Dimethyl-(2,2,3-trimethyl-3-cyclopenten-1-yl)-5-hexen-2-on

Dieses Beispiel veranschaulicht die Alkylierung des Ketons gemäß Beispiel 1 mit Methyljodid.

Ausführung: In einem trockenen 1-1-Reaktor wurden unter Durchleiten von trockenem Stickstoff 93,3 g (1,72 mol) Kaliumhydroxid-Pulver in 100 ml absolutem Toluol suspendiert und 0,83 g des Kronenethers 18-Krone-6 zugegeben. Anschließend wurden 177 g des in Beispiel 1 beschriebenen Ketons (0,86 mol) zugegeben und zu der gerührten Mischung innerhalb von 3 Stunden 273,6 g (1,9 mol) Methyljodid zudosiert. Nach der Zugabe wurde bei Raumtemperatur über Nacht weitergerührt.

Aufarbeitung: Zur Aufarbeitung wurde auf 1 l Eiswasser gegossen und die Toluolphase abgetrennt. Die Wasserphase wurde mit insgesamt 300 ml Ether extrahiert. Die vereinigten organischen Phasen wurden mit 10%iger Salzsäure und Kochsalzlösung ausgewaschen, über Natriumsulfat getrocknet, filtriert, eingeengt und über Kugelrohr vordestilliert. Die weitere Feinfraktionierung an einer Drehbandkolonne ergab 57,6 g Produkt vom Siedepunkt 75-77 °C/0,02 mbar (Ausbeute: 29% d.Theorie). Das IR-Spektrum (Film auf NaCl) zeigte Banden bei 2956, 1717 (C=O), 1638 (C=C), 1460, 1360, 1068 und 894 $cm^{-1}$.

Geruch: Bergamotte-, Rhabarber-Note, holzig.

**Beispiel 3:** 3,3-Dimethyl-(2,2,3-trimethyl-3-cyclopenten-1-yl)-5-hexen-2-ol

Dieses Beispiel veranschaulicht die selektive Reduktion der Carbonylgruppe nach der Alkylierung des Ketons gemäß Beispiel 2 zur OH-Funktion.

Ausführung: In einem 1 l Dreihalskolben wurden 15 g (0,4 mol) Natriumboranat in 200 ml Ethanol vorgelegt. Zu dieser Suspension wurden anschließend innerhalb von 2 Stunden eine Lösung von 117 g (0,5 mol) des Ketons gemäß Beispiel 2 zudosiert und die Mischung eine weitere Stunde nachgerührt.

Aufarbeitung: Zur Zerstörung überschüssigen Natriumboranats wurden 150 ml Wasser zugegeben und die Mischung über Nacht gerührt. Anschließend wurde mit Ether extrahiert, die Etherphasen mit Wasser neutral gewaschen und über Kaliumcarbonat getrocknet. Nach Filtrieren und Einengen wurde das Produkt über Kugelrohr vordestilliert und das erhaltene Rohprodukt (96 g) anschließend an einer Drehbandkolonne fraktionierend destilliert. Die Hauptmenge von 68 g ging bei Kopftemperaturen von 87-95 °C/0,1 mbar über (37,5 % der Theorie). Das Gaschromatogramm zeigte ein Isomerengemisch, wobei die beiden Hauptpeaks 65% des Gemisches ausmachten.

Charakterisieruno: Das IR-Spektrum (Film auf NaCl) zeigte Banden bei 3367 (OH), 2958, 1637 (C=C), 1462, 1375, 1361 und 891 $cm^{-1}$.

Geruch: Sandelholz.

Beispiel 4: 2,2-Dimethyl-4-(2,2,3-trimethyl-3-cyclopenten-1-yl)-4-penten-1-al

Dieses Beispiel veranschaulicht die Umsetzung eines Allylalkohols mit einem Aldehyd. Bei dieser Variante werden solche Verbindungen (I) erhalten, bei denen X eine Gruppe CO und $R^4$ Wasserstoff ist.

Ausführung: In einem 250 ml Hubrührautoklaven wurden 30 g (0,18 mol) 2-(2,2,3-Trimethyl-3-cyclopenten-1-yl)-2-propen-1-ol (hergestellt aus α-Campholenaldehyd durch Kondensation mit Formaldehyd und Reduktion der Aldehydgruppe) mit 72 g (1 mol) Isobutyraldehyd und 1,5 g Pivalinsäure vorgelegt. Der Autoklav wurde mit Stickstoff gespült und dann verschlossen. Es wurde auf 190 °C erhitzt, wobei sich ein Druck von 18 bar einstellte. Anschließend erhitzte man 7 Stunden unter diesen Bedingungen.

Aufarbeitung: Man ließ abkühlen, destillierte den überschüssigen Isobutyraldehyd ab und destillierte den Rückstand von 60 g an einer 12 cm Vigreux-Kolonne. Dabei wurden 21 g Destillat erhalten, die an einer Drehbandkolonne weiter aufgereinigt wurden. Die Hauptfraktion von 12,1 g (28% der Theorie) ging bei einer Temperatur von 78-80 °C/0,04 mbar über; die GC-Reinheit betrug 84%.

Charakterisierung: Das IR-Spektrum (Film auf NaCl) zeigte Banden bei 2958, 2928 (H-CO), 1726 (CH=O), 1636 (C=C), 1467, 1362, 1194 und 900 $cm^{-1}$. Geruch: blumig, fruchtig, holzig, Sandel-Note.

Beispiel 5: 2,2-Dimethyl-4-(2,2,3-trimethyl-3-cyclopenten-1-yl)-4-penten-1-ol

Dieses Beispiel veranschaulicht die selektive Reduktion der Carbonylgruppe des Aldehyds gemäß Beispiel 4 zur OH-Funktion.

Ausführung: In einem 250 ml Dreihalskolben wurden 2 g (0,05 mol) Natriumboranat in 100 ml Ethanol vorgelegt. Zu dieser Suspension wurde anschließend kontinuierlich eine Lösung von 15 g (0,068 mol) des Aldehyds gemäß Beispiel 4 zudosiert und die Mischung nach Abklingen der schwach exothermen Reaktion eine weitere Stunde nachgerührt.

Aufarbeitung: Die Reaktionsmischung wurde auf Ammoniumchloridlösung/Eis gegossen und mit Ether extrahiert. Die organischen Phasen wurden über Natriumsulfat getrocknet, filtriert und eingeengt. Nach einer Vordestillation über Kugelrohr (Luftbadtemperatur: 140 °C) wurde das Rohprodukt weiter über eine Drehbandkolonne aufgereinigt. Die Hauptmenge von 5,9 g wurde bei Kopftemperaturen von 82-84 °C/0,05 mbar isoliert (39 % der Theorie; GC-Reinheit: 95%).

Charakterisierung: Das IR-Spektrum (Film auf NaCl) zeigte Banden bei 3368 (OH), 2955, 1634 (C=C), 1464, 1384, 1045 (C-O), 898 und 803 $cm^{-1}$. Geruch: fruchtig, Sandelholz.

**Beispiel** 6 3,3-Dimethyl-5-(2,2,3-trimethyl-cyclopent-3-en-1-yl)-hex-5-en-2-ol

Ausführung: In einem trockenen 500 ml Dreihalskolben wurden unter Stickstoff 3,6 g (0,15 mol) Magnesium-Späne (99,5 %, Riedel de Haen) in 100 ml wasserfreiem Ether suspendiert und 21,6 g (0,15 mol) Jodmethan (99 %, Fa. Fluka) so zugetropft, daß nach Anspringen der Reaktion die Mischung unter ständigem Rückfluß gehalten wurde (ca. 25 Min.). Zu dem so zubereiteten Grignard-Reagens wurde unter Feuchtigkeitsausschluß eine Lösung von 22,0 g (0,1 mol) des nach Beispiel 4 hergestellten Aldehyds (71 %-ig) in 100 ml Ether kontinuierlich zugetropft, so daß die Reaktionsmischung während des Zutropfens durch die freiwerdende Reaktionswärme am Sieden gehalten wurde (ca. 20 Minuten). Nach der Zugabe wurde des Gemisch noch 4 Stunden bei Raumtempertur gerührt.

Aufarbeitung: Die Mischung wurde auf 500 ml eiskalte gesättigte Ammoniumchlorid-Lösung gegossen und 30 Minuten gerührt. Die Etherphase wurde abgetrennt und die Wasserphase mehrmals mit Ether extrahiert. Die vereinigten Etherphasen wurden mit gesättigter Kochsalzlösung neutral gewaschen, über Kaliumcarbonat getrocknet, eingeengt und über Kugelrohr destilliert. Das Destillat von 20 g (GC-Reinheit: 85 %) wurde an einer Drehbandkolonne fraktioniert. Ein Hauptlauf von 12 g (71 % der Theorie) ging bei Kopftemperaturen von 104-106 °C/0,05 mbar über (GC-Reinheit des Diastereomerengemisches: 99,7 %).

Analytik: IR-Spektrum (Film auf NaCl) zeigt Absorptionsbanden bei 3387 (-OH), 3075, 3033, 2958, 1633 (C=C), 1463, 1383, 1361 (gem. Dimethyl), 1092 (C-O) und 898 $cm^{-1}$.

Geruch: holzig, fruchtig, Sandelholz

**3. Kompositionsbeispiel 1**

| Tabak-Base | Gewichtsteile |
|---|---|
| Benzylacetat | 150 |
| Atrinon (Henkel) | 150 |
| Geraniol | 120 |
| Vetiverylacetat brut | 100 |
| Cedrenol | 50 |

(fortgesetzt)

| Tabak-Base | Gewichtsteile |
|---|---|
| Cedernholzöl Florida | 50 |
| DPG | 40 |
| Eichenmoos res. | 40 |
| Guajylacetat | 30 |
| Cyclamber (Henkel) | 30 |
| Isoraldein 70 | 30 |
| Benzophenon | 30 |
| Patchouliöl | 30 |
| Olibanum res. | 25 |
| Cyclohexylsalicylat (Henkel) | 25 |
| Bergamotteöl | 20 |
| Lavendelöl | 20 |
| beta-Naphthylmethylketon | 20 |
| Cumarin | 20 |
| Vanillin | 10 |
| Cinnamylacetat | 10 |
| | 1000 |

Ersatz der 40 Teile Dipropylenglykol (DPG) in obiger Mischung durch 40 Teile 3,3-Dimethyl-5-(2,2,3-trimethyl-cyclpent-3-en-1-yl)-hex-5-en-2-ol (Beispiel 6) zu der Mischung verbindet holzige und herb-würzige Aspekte dieser Base in idealer Weise und verleiht der Komposition eine Moschus-Note. Diese Effekte lassen sich weder durch herkömmliche Sandel-Noten noch durch die bekannten synthetischen Moschus-Riechstoffe erreichen.

**4. Kompositionsbeispiel 2**

| Phantasie-Fougere | Gewichtsteile |
|---|---|
| Boisambrene forte (Henkel) | 150 |
| Bergamotteöl marok. | 100 |
| Lavandinöl grosso | 100 |
| Jasmacyclat (Henkel) | 100 |
| Citronellol rein | 80 |
| Isoraldein 70 | 80 |
| Citral | 50 |
| DPG | 45 |
| Rosmarinöl | 40 |
| Eichenmoos abs. | 30 |
| Orangenöl hell | 30 |
| Linalool | 30 |
| Ylang Ylang IP | 30 |
| Vetiveröl | 20 |
| Geraniumöl Bourbon | 20 |
| Lavendel abs. | 20 |
| Zimtblätteröl | 15 |
| Korianderöl | 15 |
| Thymianöl | 15 |
| Patchouliöl | 10 |
| Ciste abs. | 10 |
| Nelkenblütenöl | 5 |

(fortgesetzt)

| Phantasie-Fougere | Gewichtsteile |
|---|---|
| Beifußöl | 5 |
| | 1000 |

Ersetzt man die 45 Teile Dipropylenglykol in obiger Formulierung durch die gleiche Menge des nach Beispiel 5 hergestellten 2,2-Dimethyl-4-(2,2,3-trimethyl-cyclopent-3-en-1-yl)-pent-4-en-1-ols, so verleiht das der Parfümerierung intensive und langanhaltende balsamisch, moschusartige Untertöne und eine fruchtige Top-Note, die die Natürlichkeit des Gesamteindrucks verstärkt.

**Patentansprüche**

1. Penten-Derivate der allgemeinen Formel (I)

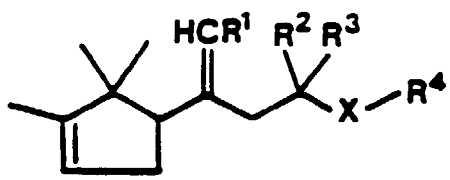

(I)

worin unabhängig voneinander der Rest $R^1$ Wasserstoff oder eine Methylgruppe, die Reste $R^2$ und $R^3$ eine Alkylgruppe mit 1 bis 5 C-Atomen, der Rest $R^4$ Wasserstoff oder eine Gruppe $CHR^5R^6$, die Reste $R^5$ und $R^6$ Wasserstoff oder eine Alkylgruppe mit 1 bis 6 C-Atomen und X eine Gruppe CO oder eine Gruppe CHOH bedeuten.

2. Penten-Derivate nach Anspruch 1, worin die Reste $R^2$ und $R^3$ Methyl oder Ethyl bedeuten.

3. Penten-Derivate nach Anspruch 2, worin der Rest $R^4$ eine Methylgruppe bedeutet.

4. Penten-Derivate nach Anspruch 2, worin der Rest $R^4$ Wasserstoff bedeutet.

5. Verfahren zur Herstellung von Penten-Derivaten der allgemeinen Formel (I)

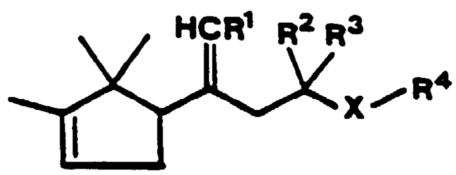

(I)

worin unabhängig voneinander der Rest $R^1$ Wasserstoff oder eine Methylgruppe, die Reste $R^2$ und $R^3$ eine Alkylgruppe mit 1 bis 5 C-Atomen, der Rest $R^4$ Wasserstoff oder eine Gruppe $CHR^5R^6$, die Reste $R^5$ und $R^6$ Wasserstoff oder eine Alkylgruppe mit 1 bis 6 C-Atomen und X eine Gruppe CO oder eine Gruppe CHOH bedeuten, durch

(a) Umsetzung von Allylalkoholen der allgemeinen Formel (II)

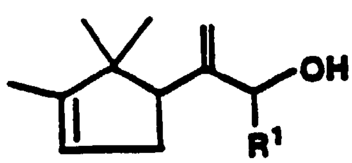

(II)

worin der Rest $R^1$ Wasserstoff oder eine Methylgruppe bedeutet, mit Aldehyden der allgemeinen Formel (III)

$$CHR^2R^3\text{-}CHO \qquad (III)$$

worin unabhängig voneinander die Reste $R^2$ und $R^3$ eine Alkylgruppe mit 1 bis 5 C-Atomen bedeuten oder
(b) Alkylierung von Verbindungen der allgemeinen Formel (IV)

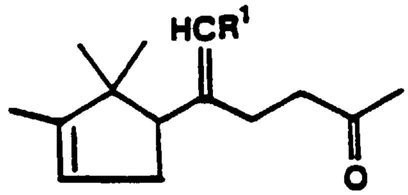

(IV)

worin der Rest $R^1$ Wasserstoff oder eine Methylgruppe bedeutet, und jeweils gegebenenfalls anschließende Reduktion der Carbonylgruppe zur OH-Funktion, z.B. mittels komplexer Hydride.

**6.** Verwendung der Penten-Derivate nach einem der Ansprüche 1 bis 5 als Riechstoffe.

**7.** Riechstoffkompositionen mit einem Gehalt an einem oder mehreren Penten-Derivaten der Formel (I) gemäß Anspruch 1 bis 6 in einer Menge von 1 - 70 Gew.-%, bezogen auf die gesamte Komposition.

**8.** Verwendung von einem oder mehreren Penten-Derivaten der Formel (I) gemäß Anspruch 1 bis 7 als Riechstoffe in kosmetischen Präparaten, technischen Produkten oder der alkoholischen Parfümerie.

**Claims**

**1.** Pentene derivatives corresponding to general formula (I):

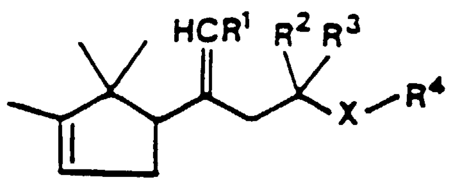

(I)

in which - independently of one another - the substituent $R^1$ is hydrogen or a methyl group, the substituents $R^2$ and $R^3$ represent an alkyl group containing 1 to 5 carbon atoms, the substituent $R^4$ is hydrogen or a group $CHR^5R^6$, where the substituents $R^5$ and $R^6$ are hydrogen or an alkyl group containing 1 to 6 carbon atoms, and X is a group CO or a group CHOH.

**2.** Pentene derivatives as claimed in claim 1, in which the substituents $R^2$ and $R^3$ are methyl or ethyl.

**3.** Pentene derivatives as claimed in claim 2, in which the substituent $R^4$ is a methyl group.

**4.** Pentene derivatives as claimed in claim 2, in which the substituent $R^4$ is hydrogen.

**5.** A process for the production of the pentene derivatives corresponding to general formula (I):

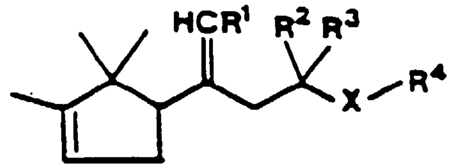

(I)

in which - independently of one another - the substituent $R^1$ is hydrogen or a methyl group, the substituents $R^2$ and $R^3$ represent an alkyl group containing 1 to 5 carbon atoms, the substituent $R^4$ is hydrogen or a group $CHR^5R^6$, where the substituents $R^5$ and $R^6$ are hydrogen or an alkyl group containing 1 to 6 carbon atoms, and X is a group CO or a group CHOH, by

(a) reaction of allyl alcohols corresponding to general formula (II):

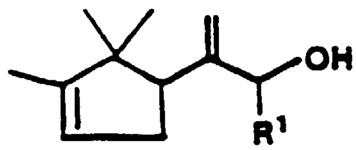

(II)

in which the substituent $R^1$ is hydrogen or a methyl group,
with aldehydes corresponding to general formula (III):

$$CHR^2R^3\text{-}CHO \quad \text{(III)}$$

in which the substituents $R^2$ and $R^3$ independently of one another represent an alkyl group containing 1 to 5 carbon atoms,
or
(b) alkylation of compounds corresponding to general formula (IV):

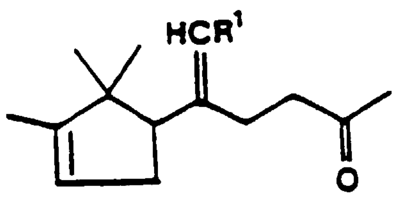

(IV)

in which the substituent $R^1$ is hydrogen or a methyl group,
and, optionally, subsequent reduction of the carbonyl group to the OH function, for example with complex hydrides.

**6.** The use of the pentene derivatives claimed in claims 1 to 5 as fragrances.

**7.** Fragrance compositions containing one or more of the pentene derivatives corresponding to formula (I) claimed in claims 1 to 6 in a quantity of 1 to 70% by weight, based on the composition as a whole.

**8.** The use of one or more of the pentene derivatives corresponding to formula (I) claimed in claims 1 to 7 as fragrances in cosmetic preparations, commercial products or alcohol-based perfumery.

## Revendications

**1.** Dérivés de pentène de formule générale (I),

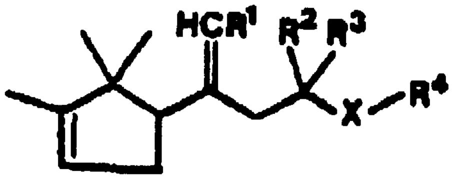

(I)

où indépendamment l'un de l'autre, le radical $R^1$ représente un hydrogène ou un groupe méthyle, les radicaux $R^2$ et $R^3$ représentent un groupe alkyle de 1 à 5 atomes de C, le radical $R^4$ un hydrogène ou un groupe $CHR^5R^6$, les radicaux $R^5$ et $R^6$ un hydrogène ou un groupe alkyle de 1 à 6 atomes de C et X un groupe CO ou un groupe CHOH.

**2.** Dérivés de pentène selon la revendication 1, où les radicaux $R^2$ et $R^3$ représentent un éthyle ou un éthyle.

**3.** Dérivés de pentène selon la revendication 2, où le radical $R^4$ représente un groupe méthyle.

**4.** Dérivés de pentène selon la revendication 2, où le radical $R^4$ représente un hydrogène.

**5.** Procédé de préparation de dérivés de pentène de formule générale (I),

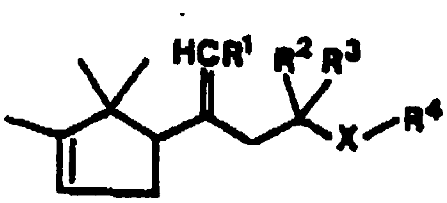

(I)

où indépendamment l'un de l'autre le radical $R^1$ représente un hydrogène ou un groupe méthyle, les radicaux $R^2$ et $R^3$ un groupe alkyle de 1 à 5 atomes de C, le radical $R^4$ un hydrogène et un groupe $CHR^5R^6$, les radicaux $R^5$ et $R^6$ un hydrogène ou un groupe alkyle de 1 à 6 atomes de C et X un groupe CO ou un groupe CHOH, par

(a) réaction d'alcools allyliques de formule générale (II),

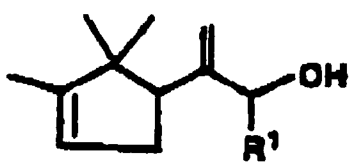

(II)

où le radical $R^1$ est un hydrogène ou un groupe méthyle, avec des aldéhydes de formule générale (III),

$$CHR^2R^3\text{-}CHO \qquad \text{(III)}$$

où indépendamment l'un de l'autre, les radicaux $R^2$ et $R^3$ représentent un groupe alkyle de 1 à 5 atomes de C, ou

b) alkylation de composés de formule générale (IV)

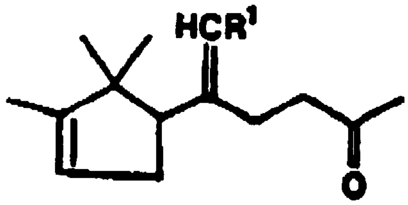

(IV)

où le radical $R^1$ représente un hydrogène ou un groupe méthyle, et respectivement le cas échéant réduction successive du groupe carbonyle en fonction OH, par exemple au moyen d'hydrures complexes.

**6.** Utilisation de dérivés de pentène selon l'une des revendications 1 à 5 comme substances odorantes.

**7.** Composition de substances odorantes contenant un ou plusieurs dérivés de pentène de formule (I) selon les revendications 1 à 6 en une quantité de 1-70 % en poids par rapport à l'ensemble de la composition.

**8.** Utilisation d'un ou plusieurs dérivés de pentène de formule (I) selon les revendications 1 à 7, comme substances odorantes dans les préparations cosmétiques, les produits industriels ou la parfumerie alcoolique.